# EUROPÄISCHE PATENTSCHRIFT

(11) **EP 0 523 534 B1**
(45) Veröffentlichungstag und Bekanntmachung des Hinweises auf die Patenterteilung: **01.02.1995**
(21) Anmeldenummer: 92111598.6
(22) Anmeldetag: 08.07.1992
(51) Int. Cl.: C07C 403/22, C07C 403/10, C07C 403/12, C07C 403/20

(54) **Verbessertes Verfahren zur Herstellung von Polyenen**
Process for the preparation of polyenes
Procédé de préparation de polyènes

(30) Priorität: 19.07.1991 DE 4123994
(43) Veröffentlichungstag der Anmeldung: 20.01.1993
(73) Patentinhaber: BASF Aktiengesellschaft, 67063 Ludwigshafen (DE)
(72) Erfinder: Jaedicke, Hagen, Dr., W-6700 Ludwigshafen (DE); Kaiser, Klaus, Dr., W-6730 Neustadt (DE); Hamm, Manfred, W-6522 Osthofen (DE)

(56) Entgegenhaltungen:
- DE-A- 2 305 267
- HELVETICA CHIMICA ACTA, Bd. 59, Nr. 2, 1976, BASEL, CH, Seiten 387 - 396; P.S. MANCHAND et al: "Synthesis of Vitamin A via Sulfones : A C15 Sulfone Route"

## Beschreibung

Die Erfindung betrifft ein verbessertes Verfahren zur Herstellung von Polyenen durch Umsetzung von α-allylischen Arylsulfonen mit α-allylischen Halogeniden oder anderen Allylierungsmitteln und anschließende Eliminierung von Arylsulfinaten.

Unter Polyenen versteht man ganz allgemein ungesättigte aliphatische Kohlenwasserstoffe mit mindestens drei oder mehr konjugierten Doppelbindungen im Molkül, d.h. Verbindungen mit zahlreichen alternierenden Einfach- und Doppelbindungen. Natürlich vorkommende farbige Polyene sind beispielsweise Lycopin und β-Carotin. Einige der natürlich vorkommenden Verbindungen sind als Lebensmittelfarbstoffe von Interesse, andere haben als biologisch medizinische Wirkstoffe Bedeutung erlangt. Die wichtigsten Polyene sind die Carotinoide, Retinoide und speziell das Vitamin A.

Entsprechend ihrer Bedeutung sind viele verschiedene Methoden zur Herstellung dieser Substanzklasse entwickelt worden (Übersicht vgl. O. Isler Carotenoids, Birkhäuser-Verlag, 1971).

Besonders bekannte Methoden zur Herstellung von Polyenen sind die Wittig-Reaktion, d.h. das Verknüpfen von α,β-ungesättigten Aldehyden mit Phosphoniumyliden (vgl. beispielsweise "Organic Reactions", Vol. 14, Verlag John Wiley, New York, 1965); die Müller-Cunradi-Reaktion, d.h. das Anlagern von Enolethern an Dialkylacetale von α,β-ungesättigten Aldehyden (vgl. beispielsweise US 2 165 962 und Chem. Abstr., 33 (1939), 8210) sowie die sogenannte Julia-Methode, d.h. das Alkylieren α-metallierter Sulfone mit Allylhalogeniden und anschließende Abspaltung eines Sulfinats (vgl. M. Julia und D. Arnould, Bull. Soc. Chim, Fr, 1973, Seiten 743 und 746). Gemäß dieser Methode wird beispielsweise von G.L. Olsen et al in "J. Org. Chem., 41 (1976), 3287, eine Synthese von all-trans-Vitamin A auf folgendem Wege beschrieben: 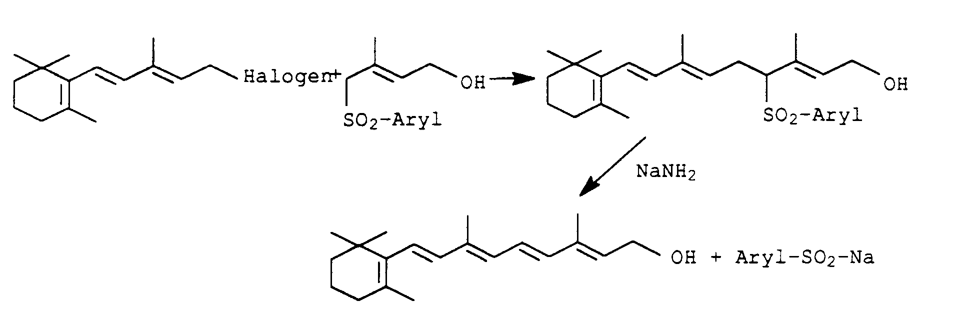
Weiterhin wird in der DOS 27 08 210 ein Verfahren zur Herstellung von Sulfonacetalen beschrieben, bei dem die Sulfone in einem mit Wasser mischbaren basischen polaren aprotischen Medium, wie Dimethylformamid oder Dimethylsulfoxid, metalliert und allyliert werden. Das dabei erhaltene Reaktionsgemisch wird anschließend hydrolysiert und das Wertprodukt in ein mit Wasser nicht mischbares Lösemittel, beispielsweise Diisopropylether extrahiert.

Alle bisher beschriebenen Verfahren zur Herstellung von Polyenen durch Alkylieren von Sulfonen weisen hinsichtlich der verwendeten Lösemittel große Nachteile auf. So wird das bei der Alkylierung erhaltene Reaktionsgemisch üblicherweise auf Eiswasser gegossen und das Wertprodukt muß aus der wäßrigen Phase isoliert werden. Hierbei geht das für die Umsetzung eingesetzte Lösemittel größtenteils verloren und führt dadurch zu einer starken Abwasserbelastung. Zur Extraktion des Wertproduktes aus der wäßrigen Phase wird zusätzlich ein nicht mit Wasser mischbares Lösungsmittel benötigt. Die weitere Umsetzung des Sulfons, vor allem die Abspaltung des Arylsulfinats erfordert wieder ein anderes Reaktionsmedium.

Beispielsweise arbeitet man bei der Herstellung von Vitamin A oder seinen Derivaten gemäß der oben beschriebenen Literaturstelle in J. Org. Chem., 41 mit einem System NaNH₂, flüssiger Ammoniak, tertiär-Butanol, oder aber in Tetrahydrofuran (THF), Lithiumdiisopropylamide. In beiden Fällen wird nach Abschluß der Reaktion auf Wasser gegossen und das Gemisch mit Diethylether extrahiert.

Es war daher die Aufgabe der Erfindung das Verfahren zur Herstellung von Polyenen durch Umsetzen von α-metallierten Sulfonen mit Allylierungsmitteln, wie insbesondere Allylhalogeniden, unter Eliminieren der gravierenden Nachteile des Standes der Technik so zu verbessern, daß es auch in technischem Maßstab vorteilhaft durchgeführt werden kann.

Es wurde nun überraschenderweise gefunden, daß man alle notwendigen Reaktionsschritte dieses Verfahrens, wie die Allylierung, eine eventuelle Wäsche des Reaktionsgemisches mit Wasser, die Eliminierung des Arylsulfinats, eine eventuell erforderliche anschließende weitere Wäsche mit Wasser und sogar eine weitere Umsetzung des erhaltenen Olefins in demselben Lösungsmittel durchführen kann, wenn man als Lösungsmittel ein inertes mit Wasser nicht mischbares polares Lösungsmittel aus der Reihe der Ketone und Ether verwendet. Überraschend war ebenfalls, daß bei dieser vorteilhaften Verfahrensführung das Hilfsreagenz Natrium-benzolsulfinat nach der Eliminierung weitgehend als Feststoff anfällt und daher besonders einfach recyclisiert werden kann.

Gegenstand der Erfindung ist dementsprechend ein Verfahren zur Herstellung von Polyenen durch die sogenannte Julia-Reaktion, d.h. die Allylierung eines mittels einer starken Base anionisierten Allyl-arylsulfons mit einem Allylierungsmittel in α-Stellung und anschließende Abspaltung der Arylsulfinsäure mit einer starken Base unter Bildung einer olefinischen Doppelbindung, das dadurch gekennzeichnet ist, daß man die Allylierung des Allyl-arylsulfons und die Abspaltung der Arylsulfinsäure im gleichen inerten, mit Wasser nicht mischbaren polaren Lösungsmittel, vorzugsweise in einem Keton der allgemeinen Formel I

R¹-CO-R² (I)

in der R¹ und R² jeweils einen geradkettigen oder verzweigten Alkylrest mit 2 bis 4 c-Atomen bedeuten oder aber R¹ und R² zusammen einen Tetramethylen- oder einen Pentamethylenrest bedeuten, durchführt.

Mit besonderem Vorteil kann das erfindungsgemäße Verfahren durchgeführt werden, wenn man die Allylierung des Allyl-arylsulfons und die Abspaltung der Arylsulfinsäure in Diethylketon, Dipropylketon, Cyclopentanon, Cyclohexanon oder Diisopropylether, vorzugsweise in Diethylketon durchführt.

Gegenstand der Erfindung ist insbesondere ein Verfahren zur Herstellung von Vitamin-A-derivaten durch Allylieren eines anionisierten Allyl-arylsulfons der Formel II 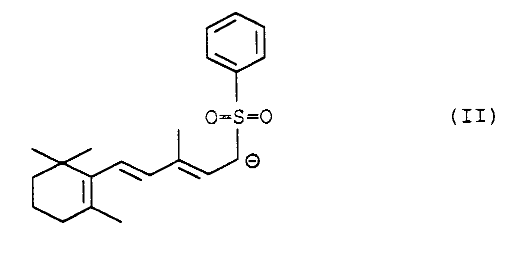
mit einer Allylverbindung der allgemeinen Formel III 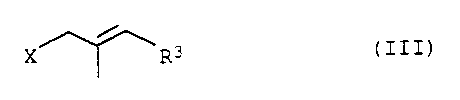
in der X eine negative Abgangsgruppe bedeutet und R³ für einen der folgenden Reste stehen kann: H, -CH₂-OCOCH₃, -COOR⁴ oder 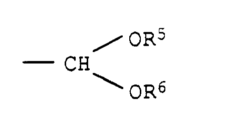
worin R⁴, R⁵ und R⁶ eine Alkylgruppe mit 1 bis 4 C-Atomen bedeuten können oder R⁵ und R⁶ zusammen einen Ethylenrest oder einen Propylenrest bedeuten, die mit einer oder mehreren Methylgruppen substituiert sein können, zu einer Arylsulfonverbindung der allgemeinen Formel IV 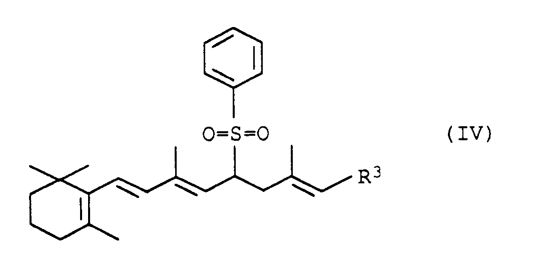
und anschließende Abspaltung der Arylsulfinsäure unter Bildung des entsprechenden Vitamin-A-derivats, das dadurch gekennzeichnet ist, daß man die Allylierung des Allyl-arylsulfons und die Abspaltung der Arylsulfinsäure in einem Keton der allgemeinen Formel I

R¹-CO-R² (I),

in der R¹ und R² jeweils einen geradkettigen oder verzweigten Alkylrest mit 2 bis 4 C-Atomen bedeuten, oder aber R¹ und R² zusammen für einen Tetramethylen- oder Pentamethylenrest steht, durchführt.

Auch das Verfahren zur Herstellung von Vitamin-A-derivaten, insbesondere von Vitamin-A-acetat, gestaltet sich besonders vorteilhaft, wenn man die Alkylierung des Allyl-arylsulfons und die Abspaltung der Arylsulfinsäure in Diethylketon durchführt.

Unter Polyenen werden im allgemeinen ungesättigte aliphatische Verbindungen verstanden, die mindestens drei konjugierte Doppelbindungen im Molekül enthalten. Die erfindungsgemäßen Verbesserungen können bei der Herstellung aller Polyene angewendet werden, die prinzipiell durch die sogenannte Julia-Methode erhältlich sind. Als nach dem erfindungsgemäßen Verfahren herstellbare Polyene seien beispielsweise genannt: β-Carotin, Astaxanthin, Canthaxantin, Neurosporaxanthin, sowie Vitamin-A-derivate, wie Vitamin-A-alkohol (Retinol), Vitamin-A-acetat, Vitamin-A-säure oder Vitamin-A-aldehyd (Retinal). Besonders vorteilhaft ist das Verfahren für die Herstellung von Vitamin-A-derivaten, insbesondere von Vitamin-A-acetat und Vitamin-A-aldehyd.

Die als Ausgangsstoffe verwendeten Allyl-arylsulfone erhält man in an sich bekannter Weise durch Umsetzen von Allylalkoholen oder Allylhalogeniden mit Salzen der Arylsulfinsäuren (vgl. beispielsweise D. Arnould et al in Bull. Soc. Chim. France 1985, II, Seite 130-31 sowie P.S. Manchand et al in Helv. Chim. Acta 59 (1976) S. 387f).

Als geeignete Allyl-arylsulfone seien beispielsweise genannt: β-Ionyliden-phenylsulfon der Formel II, Retinyl-phenylsulfon oder 3-(5,5-Dimethyl-1,3-dioxan-2-yl)-crotyl-phenylsulfon.

Als Allylverbindungen der allgemeinen Formel III 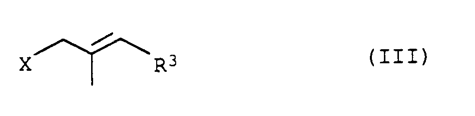
kommen alle Verbindungen dieser Formel in Betracht, in denen X für eine negative Abgangsgruppe steht und R³ die oben angegebene Bedeutung hat.

Als negative Abgangsgruppe versteht man solche Reste, die bei Einwirkung einer Base in ein Säureanion übergehen können. Beispiele für solche Gruppen sind vor allem Halogen, wie insbesondere Chlorid und Bromid; Acyloxygruppen, wie die Acetoxygruppe, die Trifluoracetoxygruppe und die Nonafluorbutoxygruppe; Sulfonyloxygruppen, wie die p-Toluolsulfonyloxygruppe und die Methansulfonyloxygruppe.

Mit besonderem Vorteil arbeitet man mit Verbindungen der Formel III, in der X für Cl oder Br steht. Genannt seien vor allem γ-Chlor-prenalneopentylglykolacetal, γ-Brom-prenal-neopentylglykolacetal, 3-(5,5-Dimethyl-1,3-dioxan-2-yl)-crotylchlorid, 3-Chlormethyl-crotonaldehydneopentylglycolacetal und Vitamin-A-acetat.

Das Anionisieren der Allyl-arylsulfone erfolgt durch Umsetzen mit einer starken Base in einem Lösungsmittel. Als geeignete starke Basen seien beispielsweise genannt: KOH, KOCH₃, NaOCH₃, NaNH₂, KO-tert.-butyl.

Hierbei ist zu beachten, daß die verwendete Base nicht einen der Reaktionspartner zersetzt. Beispielsweise bei der Allylierung mit γ-Brom-Prenylacetat darf kein KOCH₃ verwendet werden, da dieses die Acetylgruppe verseifen würde und das sich bildende Prenol sich durch Ringschlußreaktion der gewünschten Umsetzung entziehen würde. Mit KO-tert.-butyl oder NaNH₂ wäre dagegen diese Umsetzung erfolgreich. Welche Base eingesetzt werden kann, kann man durch einfache Vorversuche feststellen.

Besonders geeignet sind Alkalialkoholate, wie KOCH₃ und KOC₂H₅, insbesondere das Kalium-tert.-butylat.

Auch die Abspaltung der Arylsulfinsäure erfolgt mit starken Basen. Geeignete starke Basen sind beispielsweise NaOH, KOH, KOCH₃, KOC₂H₅ oder KO-tert.-butyl.

Mit besonderem Vorteil verwendet man Alkalialkoholate, insbesondere - wenn möglich - die gleichen, wie sie für die Anionisierung der Allyl-arylsulfone verwendet wurden.

So verwendet man beispielsweise bei der erfindungsgemäßen Herstellung von Vitamin-A-acetat aus dem β-Ionyliden-phenylsulfon und γ-Brom-prenylacetat beim Anionisieren des β-Ionylidenylphenylsulfons Kalium-tert.-butanolat oder NaNH₂, später beim Eliminieren der Arylsulfinsäure dagegen Alkalimethylate oder Alkalihydroxide. Dennoch können beide Stufen als Eintopfreaktion durchgeführt werden.

Besonders vorteilhaft gestaltet sich das erfindungsgemäße Verfahren bei der Herstellung von Retinal, da hierbei in beiden Stufen sehr vorteilhaft KOCH₃ oder NaOCH₃, vorzugsweise KOCH₃ als Base eingesetzt werden kann.

Beide Reaktionsschritte werden erfindungsgemäß in dem gleichen inerten, mit Wasser nicht mischbaren Lösungsmittel durchgeführt. Als geeignete Lösungsmittel seien beispielsweise folgende genannt: Ketone, wie 4-Heptanon (Dipropylketon), 3-Pentanon (Diethylketon), Cyclopentanon und Cyclohexanon und Ether, wie Diisopropylether.

Mit besonderem Vorteil verwendet man Diethylketon. Methylketone dagegen sind nicht geeignet.

Zur Durchführung des erfindungsgemäßen Verfahrens geht man im allgemeinen so vor, daß man eine Lösung des Allyl-phenylsulfons und des Allylierungsmittels vorlegt und hierzu die geeignete starke Base addiert.

Mit Hilfe des erfindungsgemäßen Verfahrens können beispielsweise als Farbstoffe oder Wirkstoffe geeignete Polyene auch in technischem Maßstab sehr vorteilhaft hergestellt werden.

### Beispiel 1

A. Herstellung der Lösung eines C₁₅-Sulfons in Diethylketon 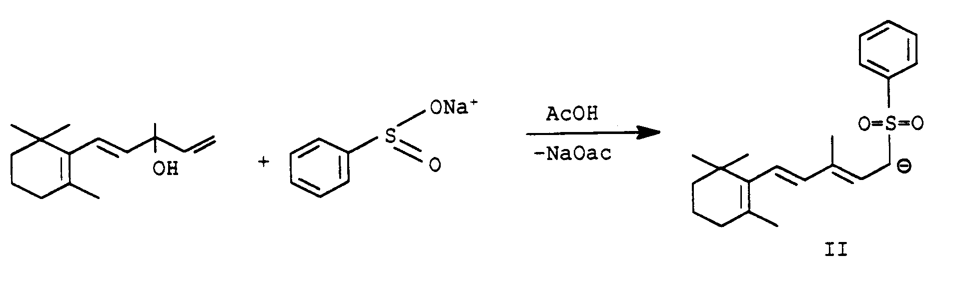 164 g (ca. 1 mol) Natriumbenzolsulfinat wurden in einem Gemisch aus 300 ml Wasser und 350 ml AcOH auf 60°C erwärmt. Bei dieser Temperatur tropfte man innerhalb von 40 Minuten (min) 200 g (92 - 93 %ig entsprechend ca. 0,87 mol) Vinylionol und rührte das Reaktionsgemisch noch 4 Stunden (h) bei 60°C. Anschließend wurden 350 ml Diethylketon (DEK) zugefügt und nach 10 min Durchmischen die gebildeten Phasen getrennt. Die vereinigten organischen Phasen wurden mit 350 ml Wasser versetzt, das unter Rühren mit konz. wäßriger NaOH auf einen pH-Wert von 8 eingestellt wurde. Das so erhaltene C₁₅-Sulfon in DEK wurde abgetrennt und über MgSO₄ getrocknet und filtriert.
B. Allylierung, Benzolsulfinat-Eliminierung und Hydrolyse in Diethylketon 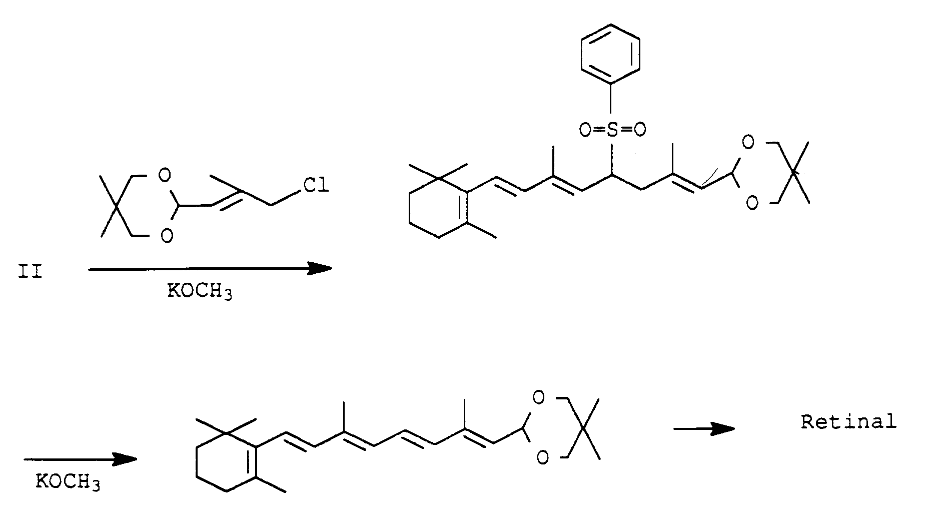 Zu der gemäß Beispiel 1A hergestellten C₁₅-Sulfon-Lösung in DEK wurden 190 g reines γ-Chlor-prenal-neopentylglykolacetal (hergestellt gemäß DE-OS 2 917 413) zugefügt und anschließend unter Kühlung auf 0°C innerhalb 1 h eine Suspension von 150 g (ca. 2,1 mol) Kaliummethylat in 350 ml DEK addiert. Nach 1 h Rühren bei Raumtemperatur (RT) wurde das Reaktionsgemisch auf 60°C erwärmt und nach weiteren 60 min Rühren das ausgefallene Gemisch aus KCl und Kaliumbenzolsulfinat mit 250 ml Wasser gelöst und die wäßrige Unterphase abgetrennt.
   Die abgetrennte organische Phase wurde mit 300 ml Isopropanol und 300 ml einer 2 %igen H₂SO₄ versetzt und 10 min bei 40°C gerührt. Nach Abkühlen auf 20°C wurde die wäßrige Phase abgetrennt und die organische Phase unter vermindertem Druck eingeengt. Man erhielt 222 g (entsprechend einer Ausbeute von 84 % der Theorie) reines Retinal, das sich zu 79 % aus der all-trans-Form, zu 18 % aus dem 13-cis-Isomeren und zu ca. 1 % aus dem 9-cis-Isomeren zusammensetzte.

### Beispiel 2

### Allylierung, Benzolsulfinat-Eliminierung und Hydrolyse in 4-Heptanon (Dipropylketon; DPK)

34,5 g reines C₁₅-Sulfon (hergestellte analog Beispiel 1A) wurden in 100 ml DPK gelöst und 23 g γ-Chlor-prenal-neopentylglykolacetal zugefügt. Anschließend wurde innerhalb von 30 min unter Kühlen auf ca. 20°C portionsweise 18 g Kaliummethylatpulver eingebracht. Nach 20 min Rühren bei RT wurde auf 55°C erwärmt und noch 2 h bei 55°C gerührt. Anschließend wurden 50 ml Wasser eingetropft, durchmischt und danach die gebildete Unterphase abgetrennt. Die abgetrennte Oberphase wurde mit 30 ml Isopropanol und 30 ml 2 %ig. H₂SO₄ versetzt, 30 min auf 70°C erwärmt und dann die Phasen getrennt. Aus der organischen Phase wurden 24,1 g Retinal in überwiegend all-trans-Form isoliert.

### Beispiel 3

Man arbeitete wie in Beispiel 2 beschrieben, verwendete jedoch 100 ml Cyclohexanon anstelle von 100 ml Dipropylketon. Man erhielt nach der Hydrolyse durch Einengen der abgetrennten organischen Phase 28,4 g eines 82 %igen rohen Retinals.

### Beispiel 4

### Recylisierung von Kaliumbenzolsulfinat

Die gemäß Beispiel 1B erhaltene wäßrige Lösung von KCl und Kaliumbenzolsulfinat wurde mit 300 ml Eisessig versetzt, das Reaktionsgemisch auf 60°C erwärmt, dann innerhalb von 25 min tropfenweise mit 178 g (0,81 mol) Vinylionol versetzt und noch 1 h bei 60°C gerührt. Anschließend wurde 3x mit 100 ml DEK extrahiert, die vereinigten Extrakte mit verdünnter Sodalösung Säure-frei gewaschen. Mittels HPLC-Untersuchung wurde eine Ausbeute von 92 % cis-trans-C₁₅-Sulfon, bezogen auf Vinylionol festgestellt.

### Beispiel 5

### Allylierung von 3-(5,5-Dimethyl-1,3-dioxan-2-yl)-crotyl-phenylsulfon (1) mit 3-(5,5-Dimethyl-1,3-dioxan-2-yl)-crotylchlorid (2)

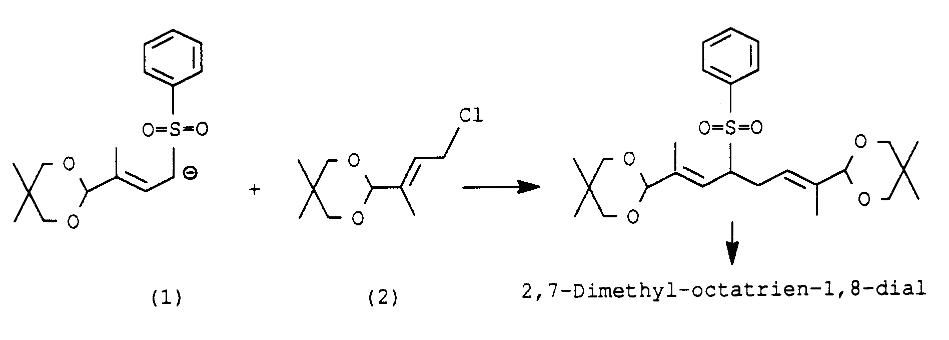

### A. Herstellung des Phenylsulfons 1

Zu einer Lösung von 52,6 g (0,25 mol) 3-(5,5-Dimethyl-1,3-dioxan-2-yl)-crotylchlorid (hergestellt gemäß Liebigs Ann. Chem. 1976, Seite 2194) in 200 ml DEK wurden 42 g (0,25 mol) Natriumbenzolsulfinat addiert, das erhaltene Reaktionsgemisch 4 h unter Rückfluß zum Sieden erhitzt, das dabei auskristallisierte NaCl abfiltriert und der Niederschlag mit 300 ml DEK gewaschen.

### B. Allylierung, Benzolsulfinat-Eliminierung und Hydrolyse

Zu der gemäß Beispiel 5A erhaltenen Lösung des Phenylsulfons 1 wurden 52,6 g (0,25 mol) 3-(5,5-Dimethyl-1,3-dioxan-2-yl)crotylchlorid (2) addiert und das Gemisch auf 0°C abgekühlt. Anschließend wurden 16,4 g pulverisiertes KOH zugefügt und noch 60 min bei 0°C gerührt. Anschließend fügte man 21 g (0,3 mol) Kaliummethylat in pulverisierter Form zu und ließ das Gemisch unter Rühren auf RT erwärmen. Nach 2 h hatte sich alles Allylierungsprodukt zu 2,7-Dimethyl-octatrien-1,8-dial-dineopentylglykolacetal umgesetzt. Man erhielt nach Wäsche des Reaktionsaustrages mit Wasser 68 g (entsprechend 81 % der Theorie) 2,7-Dimethyl-octatrien-1,8-dialdineopentylglykolacetal (3) überwiegend in der trans-Form. Das Diacetal (3) wurde in einem Zweiphasensystem mit DEK und 200 ml verdünnter H₂SO₄ vollständig zu 2,7-Dimethyl-octatrien-1,8-dial verseift. Das Ziel wurde in einer Ausbeute von 79 %, bezogen auf 3-(5,5-Dimethyl-1,3-dioxan-2-yl)-crotylchlorid isoliert.

### Beispiel 6

### Allylierung von 3-(5,5-Dimethyl-1,3-dioxan-2-yl)-crotyl-phenylsulfon mit 3-Chlormethyl-crotonaldehydneopentylglykolacetal.

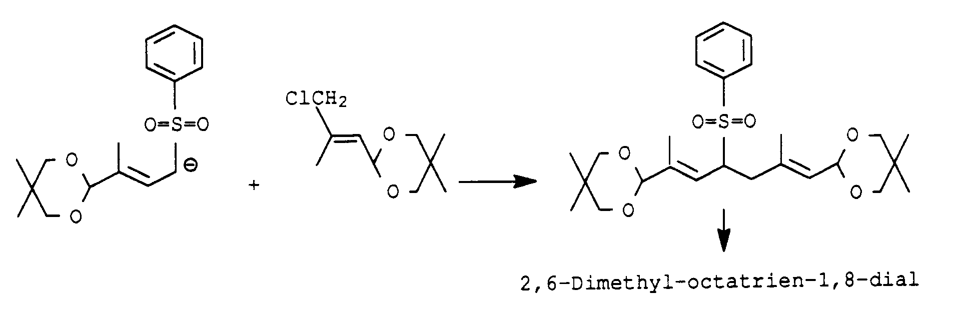

Zu einer analog Beispiel 5A hergestellten Lösung von 31,0 g (0,1 mol) 3-(5,5-Dimethyl-1,3-dioxan-2-yl)-crotyl-phenylsulfon in 200 ml DEK wurden 20,5 g (0,1 mol) 3-Chlormethyl-crotonaldehyd-neopentylglykolacetal addiert. Anschließend wurde unter Rühren auf 0°C abgekühlt, und dann innerhalb von 20 min 17 g (0,24 mol) festes Kaliummethylat zugegeben. Nach 1 h ließ man das Reaktionsgemisch auf RT erwärmen und rührte dann 2 h. Mittels HPLC-Kontrollen wurde nachgewiesen, daß 30,6 g (entsprechend 91 % der Theorie) 2,7-Bis-(5,5-dimethyl-1,3-dioxan-2-yl)-6-methyl-hepta-2,4,6-trien entstanden waren. Das Reaktionsgemisch wurde mit wenig Wasser gewaschen und zu der isolierten DEK-Lösung des Wertprodukts 60 ml Isopropanol und 60 ml 2 %iger H₂SO₄ und 17,4 g Propionaldehyd zwecks Umacetalisierung addiert. Nach 2 h Rühren bei 40°C waren die Acetalgruppen vollständig hydrolysiert. Anschließend wurde die wäßrige Phase abgetrennt und die organische Phase eingeengt. Nach Zugabe von 50 ml Isopropanol zum Rückstand kristallisierten 11,1 g (entsprechend 75 % d. Th.) an 2,6-Dimethyl-octatrien-1,8-dial vom Fp. 99-100°C aus.

### Beispiel 7

### Allylierung des C₁₅-Sulfons gemäß Beispiel 1A mit γ-Brom-prenylacetat

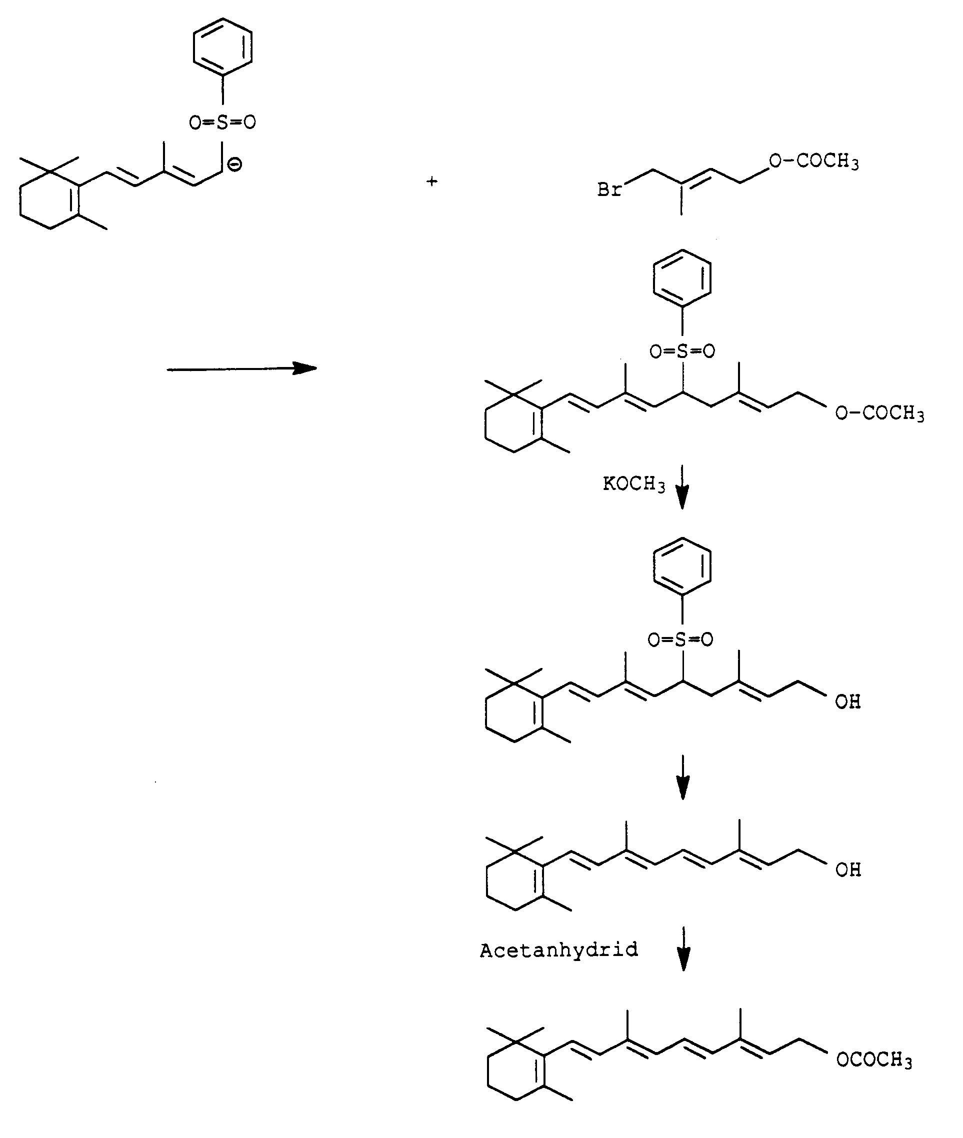

Zu 34,5 g (0,1 mol) eines gemäß Beispiel 1A hergestellten C₁₅-Sulfons in 200 ml DEK wurden 22 g γ-Brom-prenylacetat (hergestellt gemäß Beispiel 1 von US 4 175 204) addiert. Anschließend kühlte man auf 0°C ab und tropfte bei dieser Temperatur in 30 min eine Lösung von 15 g (ca. 0,13 mol) Kalium-tert.-butanolat in 100 ml DEK zu. Nach weiteren 60 min gab man 17 g (0,24 mol) Kaliummethylat zu und erwärmte auf 70°C. Nach 4-stündigem Rühren wurden 200 ml Wasser zugegeben und die Unterphase abgetrennt. Zur Oberphase gab man 30,6 g (0,3 mol) Acetanhydrid und 3 Tropfen conc. H₂SO₄. Nach 60 min war aller Vitamin-A-alkohol verestert. Man wusch mit verdünnter Sodalösung säurefrei. In der DEK-Phase hatten sich 26,9 g (82 % d. T.) Vitamin-A-acetat überwiegend in der trans-Form gebildet. Man isolierte das Wertprodukt durch schonendes Abdampfen des Lösungsmittels und Kristallisation aus Methanol.

### Beispiel 8

### Allylierung von β-Ionylidenethyl-phenylsulfon mit γ-Chlor-prenal-neopentylglykolacetal

34,5 g β-Ionylidenethyl-phenylsulfon (C₁₅-Sulfon; hergestellt nach D. Arnould et al. in Bull. Soc. Chim. France 1985, II-130) und 22 g γ-Chlor-prenal-neopentylglykolacetal wurden in 300 ml Diisopropylether gelöst, die Lösung auf 0°C gekühlt und bei dieser Temperatur innerhalb 1 h portionsweise mit 21 g pulverisiertem Kaliummethylat versetzt.

Anschließend wurde das Reaktionsgemisch noch 1 h bei RT gerührt und dann auf 60°C erwärmt. Nach 1 h hatte sich das Phenylsulfon vollständig abgespalten. Mit 50 ml Wasser wurden das gebildete Kaliumsulfinat und KCl vollständig aus der organischen Phase herausgelöst. Die wäßrige Phase ließ sich problemlos zur erneuten Herstellung von C₁₅-Sulfon aus Vinylionol einsetzen. Die organische Phase mit dem Alkylierungs-/Eliminierungsprodukt wurde mit 30 ml Isopropanol, 30 ml 2 %iger Schwefelsäure und 6 g Propanal versetzt. Nach 20 min bei 60°C war das Acetal vollständig verseift. Man trennte die wäßrige Phase ab, wusch die organische zweimal mit verdünnter NaHCO₃-Lösung und mit Wasser. Nach Trocknen über Na₂SO₄ erhielt man 279 g einer Lösung von 25,3 g Retinal in Diisopropylether entsprechend einer Ausbeute von 89 % der Theorie.

## Patentansprüche

1. Verbessertes Verfahren zur Herstellung von Polyenen durch die sogenannte Julia-Reaktion, d.h. die Allylierung eines anionisierten Allyl-arylsulfons mit einer Allylverbindung in α-Stellung und anschließende Abspaltung der Arylsulfinsäure mittels einer starken Base unter Bildung einer olefinischen Doppelbindung, dadurch gekennzeichnet, daß man die Allylierung des Allyl-arylsulfones und die Abspaltung der Arylsulfinsäure im gleichen inerten, mit Wasser nicht mischbaren polaren Lösungsmittel aus der Reihe der Ketone und Ether durchführt.

2. Verfahren gemäß Anspruch 1, dadurch gekennzeichnet, daß man die Allylierung des Allyl-arylsulfons und die Abspaltung der Arylsulfinsäure in einem Keton der allgemeinen Formel I
R¹-CO-R² (I)
in der R¹ und R² jeweils einen geradkettigen oder verzweigten Alkylrest mit 2 bis 4 Kohlenstoffatomen bedeuten oder aber R¹ und R² zusammen einen Tetramethylen- oder einen Pentamethylenrest bedeuten, durchführt.

3. Verfahren gemäß Anspruch 1, dadurch gekennzeichnet, daß man die Allylierung des Allyl-arylsulfons und die Abspaltung der Arylsulfinsäure in Diethylketon, Dipropylketon, Cyclopentanon, Cyclohexanon oder Diisopropylether durchführt.

4. Verfahren gemäß Anspruch 2, dadurch gekennzeichnet, daß man die Allylierung des Allyl-arylsulfons und die Abspaltung der Arylsulfinsäure in Diethylketon durchführt.

5. Verfahren gemäß Anspruch 1 zur Herstellung von einem Vitamin-A-derivat durch Allylieren eines anionisierten Allyl-arylsulfons der Formel II 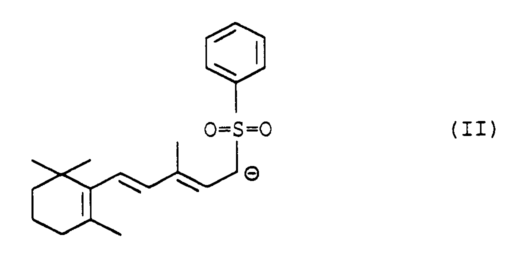 mit einer Allylverbindung der allgemeinen Formel III 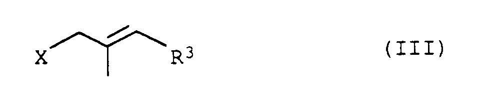 in der X eine negative Abgangsgruppe bedeutet und in der R³ für einen der folgenden Reste stehen kann: H, -CH₂-OCOCH₃, -COOR⁴ oder 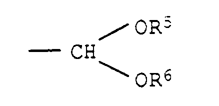 worin R⁴, R⁵ und R⁶ eine Alkylgruppe mit 1 bis 4 C-Atomen bedeuten können oder R⁵ und R⁶ zusammen einen Ethylenrest oder einen Propylenrest bedeuten, die mit einer oder mehreren Methylgruppen substituiert sein können, zu einer Arylsulfonverbindung der allgemeinen Formel IV 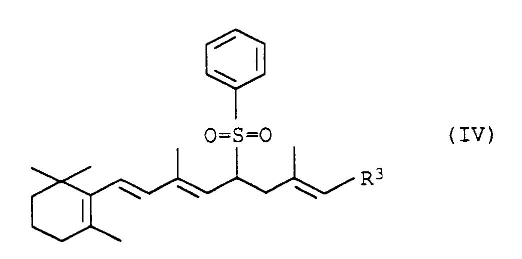 und anschließende Abspaltung der Arylsulfinsäure unter Bildung des entsprechenden Vitamin-A-derivats, dadurch gekennzeichnet, daß man die Allylierung des Allyl-arylsulfons und die Abspaltung der Arylsulfinsäure in einem Keton der allgemeinen Formel I
R¹-CO-R² (I),
in der R¹ und R² jeweils einen geradkettigen oder verzweigten Alkylrest mit 2 bis 4 C-Atomen bedeuten, oder aber R¹ und R² zusammen für einen Tetramethylen- oder Pentamethylenrest bedeuten, durchführt.

6. Verfahren gemäß Anspruch 5, dadurch gekennzeichnet, daß man die Allylierung des Allyl-arylsulfons und die Abspaltung der Arylsulfinsäure in Diethylketon durchführt.

7. Verfahren gemaß Anspruch 5, dadurch gekennzeichnet, daß man die Allylierung des Allyl-arylsulfons mit einer Allylverbindung der allgemeinen Formel III durchführt 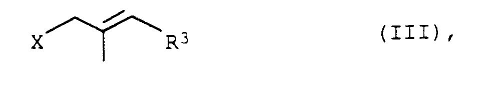 in der X für Cl oder Br und R³ für -CH₂-OCOCH₃ oder 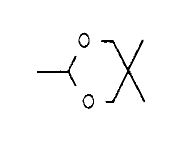 steht.

8. Verfahren gemäß Anspruch 5 zur Herstellung eines Retinalacetals durch Allylieren eines anionisierten Allylarylsulfons der Formel II 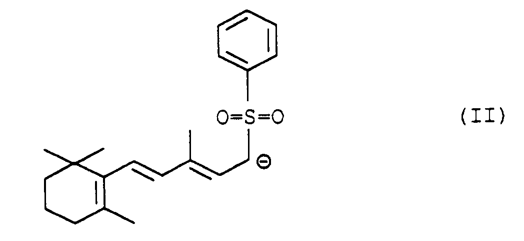 mit einer Allylverbindung der allgemeinen Formel III 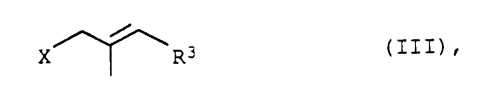 in der X für Cl oder Br steht und R³ für eine Acetalgruppe 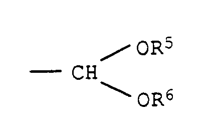 in der R⁵ und R⁶ die in Anspruch 5 angegebene Bedeutung haben, zu einer Allyl-arylsulfonverbindung der allgemeinen Formel IV 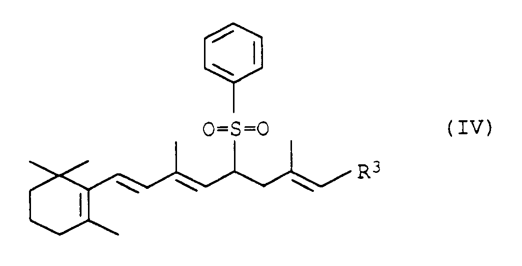 und anschließende Abspaltung der Arylsulfinsäure unter Bildung des entsprechenden Retinalacetals, dadurch gekennzeichnet, daß man die Allylierung des Allyl-arylsulfons und die Abspaltung der Arylsulfinsäure mit Kaliummethylat in Diethylketon durchführt.

9. Verfahren gemäß Anspruch 5 zur Herstellung von Vitamin-A-acetat durch Allylieren eines anionisierten Allyl-arylsulfons der Formel II 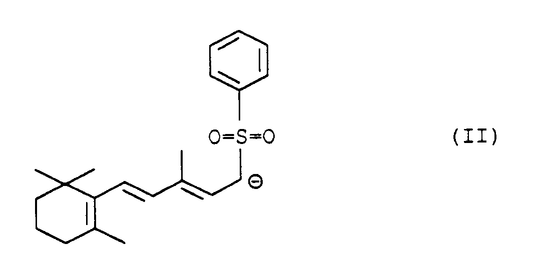 mit einer Allylverbindung der allgemeinen Formel III 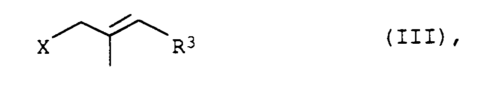 in der X für Cl oder Br steht und R³ für -CH₂-OCOCH₃ steht, zu einer Allyl-arylsulfonverbindung der allgemeinen Formel IV 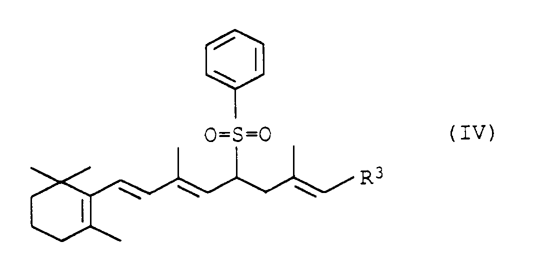 und anschließende Abspaltung der Arylsulfinsäure unter Bildung von Vitamin-A-acetat, dadurch gekennzeichnet, daß man die Allylierung des Allyl-arylsulfons und die Abspaltung der Arylsulfonsäure in Diethylketon durchführt, wobei bei der Allylierung Kalium-tert.-butylat und bei der Abspaltung der Arylsulfinsäure Alkalihydroxide oder Alkalimethylate als starke Basen verwendet werden.

## Claims

1. An improved process for the preparation of polyenes by the Julia reaction, ie. the allylation of an anion of an allyl aryl sulfone with an allyl compound in the α position and subsequent elimination of the arylsulfinic acid using a strong base to form an olefinic double bond, which comprises carrying out the allylation of the allyl aryl sulfone and the elimination of the arylsulfinic acid in the same inert, polar solvent, from the series comprising ketones and ethers, which is immiscible with water.

2. A process as claimed in claim 1, wherein the allylation of the allyl aryl sulfone and the elimination of the arylsulfinic acid are carried out in a ketone of the formula I
R¹-CO-R² (I)
where R¹ and R² are each straight-chain or branched alkyl of 2 to 4 carbons, or R¹ and R² together are tetramethylene or pentamethylene.

3. A process as claimed in claim 1, wherein the allylation of the allyl aryl sulfone and the elimination of the arylsulfinic acid are carried out in diethyl ketone, dipropyl ketone, cyclopentanone, cyclohexanone or diisopropyl ether.

4. A process as claimed in claim 2, wherein the allylation of the allyl aryl sulfone and the elimination of the arylsulfinic acid are carried out in diethyl ketone.

5. A process as claimed in claim 1 for the preparation of a vitamin A derivative by allylation of an anion of an allyl aryl sulfone of the formula II 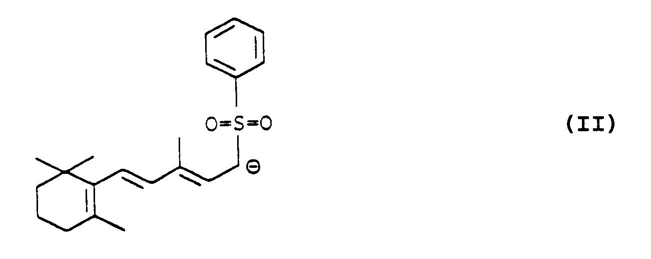 with an allyl compound of the formula III 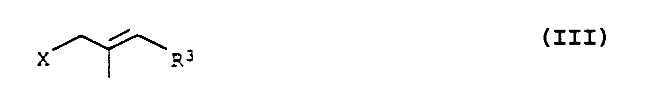 where X is a negative leaving group and where R³ is H, -CH₂-OCOCH₃, -COOR⁴ or 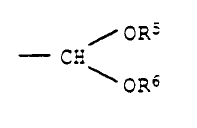 where R⁴, R⁵ and R⁶ are each alkyl of 1 to 4 carbons, or R⁵ and R⁶ together are ethylene or propylene which can be substituted by one or more methyl groups, to give an aryl sulfone of the formula IV 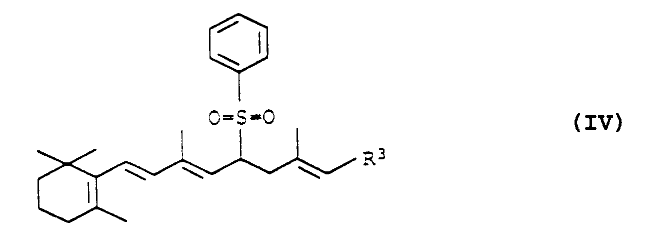 and subsequent elimination of the arylsulfinic acid to form the corresponding vitamin A derivative, which comprises carrying out the allylation of the allyl aryl sulfone and the elimination of the arylsulfinic acid in a ketone of the formula I
R¹-CO-R² (I)
where R¹ and R² are each straight-chain or branched alkyl of 2 to 4 carbon atoms, or R¹ and R² together are tetramethylene or pentamethylene.

6. A process as claimed in claim 5, wherein the allylation of the allyl aryl sulfone and the elimination of the arylsulfinic acid are carried out in diethyl ketone.

7. A process as claimed in claim 5, wherein the allylation of the allyl aryl sulfone is carried out with an allyl compound of the formula III 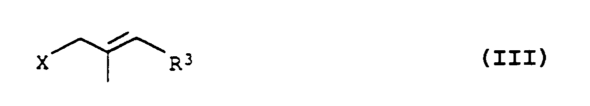 where X is Cl or Br and R³ is -CH₂-OCOCH₃ or

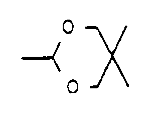

8. A process as claimed in claim 5 for the preparation of a retinal acetal by allylation of an anion of an allyl aryl sulfone of the formula II 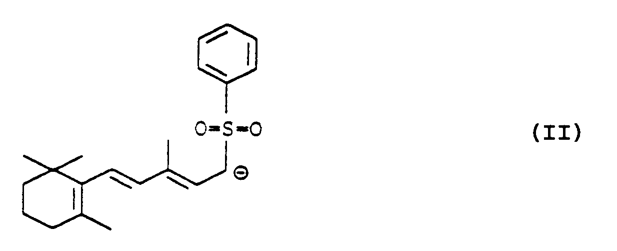 with an allyl compound of the formula III 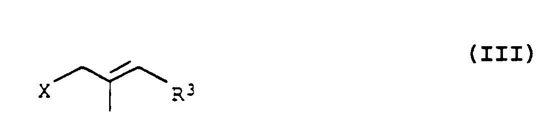 where X is Cl or Br and R³ is 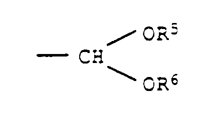 where R⁵ and R⁶ have the meaning stated in claim 5, to give an allyl aryl sulfone of the formula IV 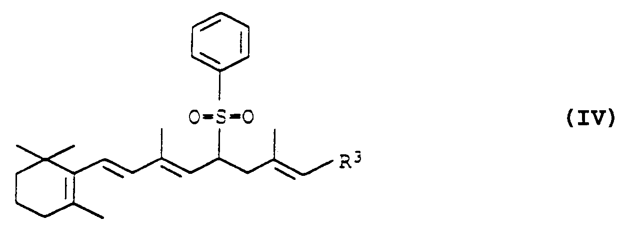 and subsequent elimination of the arylsulfinic acid to form the corresponding retinal acetal, which comprises carrying out the allylation of the allyl aryl sulfone and the elimination of the arylsulfinic acid with potassium methoxide in diethyl ketone.

9. A process as claimed in claim 5 for the preparation of vitamin A acetate by allylation of an anion of an allyl aryl sulfone of the formula II 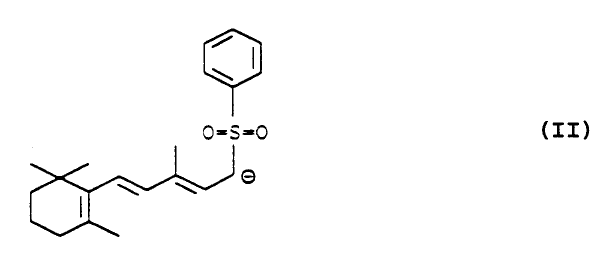 with an allyl compound of the formula III 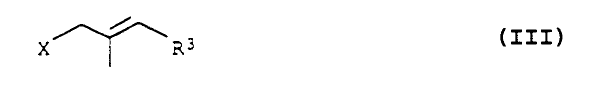 where X is Cl or Br and R³ is -CH₂-OCOCH₃, to give an allyl aryl sulfone of the formula IV 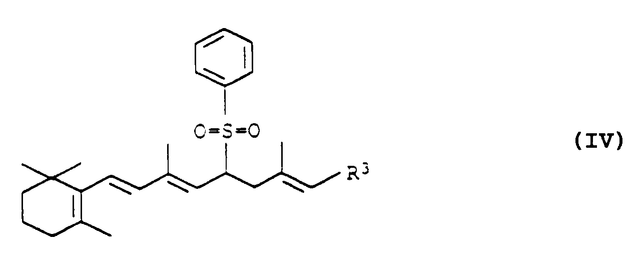 and subsequent elimination of the arylsulfinic acid to form vitamin A acetate, which comprises carrying out the allylation of the allyl aryl sulfone and the elimination of the arylsulfinic acid in diethyl ketone, the strong bases used being potassium tert-butoxide for the allylation and alkali metal hydroxides or alkali metal methyoxides for the elimination of the arylsulfinic acid.

## Revendications

1. Procédé amélioré de préparation de polyènes par la réaction de Julia, c'est-à-dire l'allylation d'une allylarylsulfone anionisée avec un composé α-allylique, suivie d'une séparation de l'acide arylsulfinique au moyen d'une base forte avec formation d'une double liaison oléfinique, caractérisé en ce que l'allylation de l'allylarylsulfone et la séparation de l'acide arylsulfinique sont menées dans un même solvant inerte polaire non miscible à l'eau de la série des cétones et des éthers.

2. Procédé selon la revendication 1, caractérisé en ce que l'allylation de l'allylarylsulfone et la séparation de l'acide arylsulfinique sont menées dans une cétone de formule générale (I)
R¹-CO-R² (I)
où R¹ et R² représentent chacun un reste alkyle à chaîne linéaire ou ramifiée avec de 2 à 4 atomes de carbone, ou bien R¹ et R² représentent ensemble un reste tétraméthylène ou pentaméthylène.

3. Procédé selon la revendication 1, caractérisé en ce que l'allylation de l'allylarylsulfone et la séparation de l'acide arylsulfinique sont menées dans la diéthylcétone, la dipropylcétone, la cyclopentanone, la cyclohexanone ou l'éther diisopropylique.

4. Procédé selon la revendication 2, caractérisé en ce que l'allylation de l'allylarylsulfone et la séparation de l'acide arylsulfinique sont menées dans la diéthylcétone.

5. Procédé selon la revendication 1 la pour la préparation d'un dérivé de la vitamine A par allylation d'une allylarylsulfone anionisée de formule II 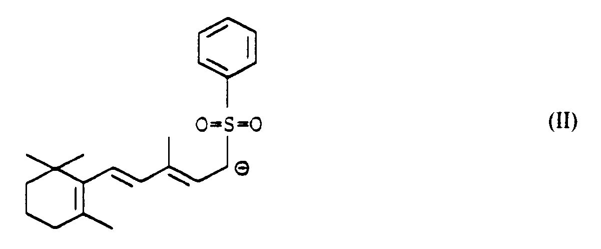 avec un composé allylique de formule générale III 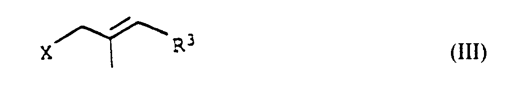 où X est un groupe terminal négatif, et où R³ peut représenter l'un des restes suivants : H, -CH₂-OCOCH₃, -COOR⁴ ou bien 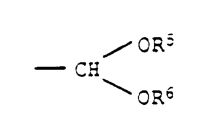 où R⁴, R⁵ et R⁶ peuvent être un groupe alkyle ayant de 1 a 4 atomes de carbone, ou R⁵ et R⁶ représentent ensemble un reste éthylène ou un reste propylène, auquel un ou plusieurs groupes mèthyle peuvent se substituer,
pour donner un composé arylsulfonique de formule générale IV 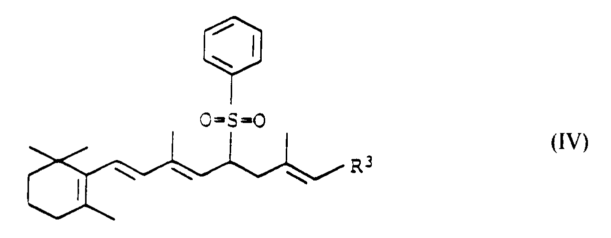 suivie d'une séparation de l'acide arylsulfinique avec formation du dérivé de la vitamine A en question, caractérisé en ce que l'allylation de l'allylarylsulfone et la séparation de l'acide arylsulfinique sont menées dans une cétone de formule générale I
R¹-CO-R² (I)
où R¹ et R² représentent chacun un reste alkyle à chaîne linéaire ou ramifiée, avec de 2 à 4 atomes de carbone, ou bien, R¹ et R² représentent ensemble un reste tétraméthylène ou pentaméthylène.

6. Procédé selon la revendication 5, caractérisé en ce que l'allylation de l'allylarylsulfone et la séparation de l'acide arylsulfinique sont menées dans la diéthylcétone.

7. Procédé selon la revendication 5, caractérisé en ce que l'allylation de l'allylarylsulfone est menée avec un composé allylique de formule générale III 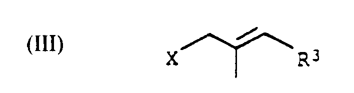 où X est Cl ou Br et R³ est -CH₂-OCOCH₃ ou

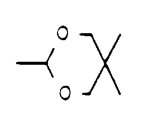

8. Procédé selon la revendication 5 pour la préparation d'un rétinalacétal par allylation d'une allylarylsulfone anionisée de formule II 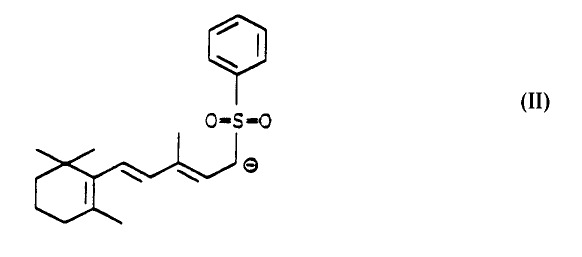 avec un composé allylique de formule générale III 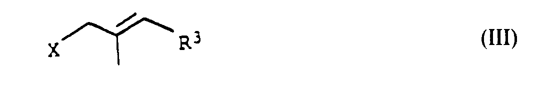 où X est Cl ou Br et R³ est un groupe acétal 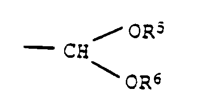 où R⁵ et R⁶ ont la signification donnée dans la revendication 5, pour donner un composé allyl-arylsulfonique de formule générale IV 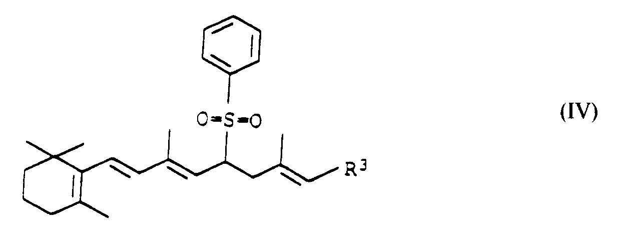 suivie de la séparation de l'acide arylsulfinique pour former le rétinalacétal en question, caractérisé en ce que l'allylation de l'allylarylsulfone et la séparation de l'acide arylsulfinique est menée en présence de méthylate de potassium dans la diéthylcétone.

9. Procédé selon la revendication 5 pour la préparation d'acétate de vitamine A par allylation d'une allylarylsulfone anionisé de formule II 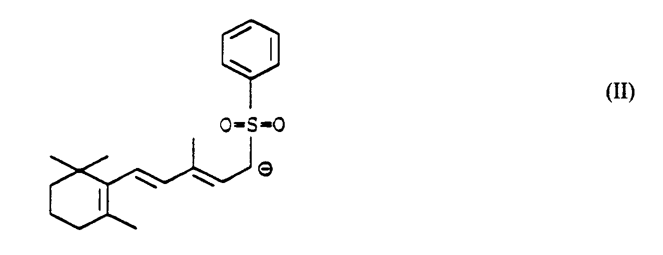 avec un composé allylique de formule générale III 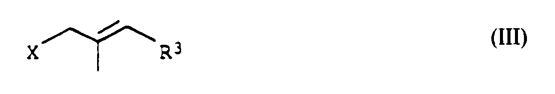 où X est Cl ou Br et R³ est -CH₂-OCOCH₃,
pour donner un composé allyl-arylsulfonique de formule générale IV 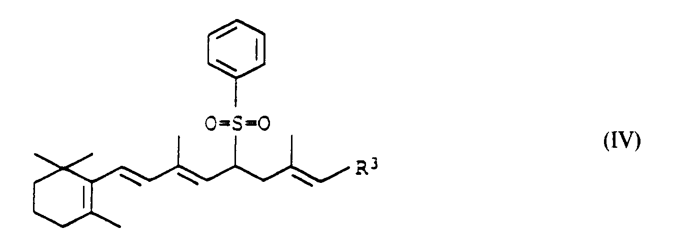 suivie de la séparation de l'acide arylsulfinique pour former de l'acétate de vitamine A. caractérisé en ce que l'allylation de l'allylarylsulfone et la séparation de l'acide arylsulfinique sont menées dans la diéthylcétone, et où on utilise comme bases fortes, pour l'allylation, le tert.-butylate de potassium et pour la séparation de l'acide arylsulfinique, des hydroxydes alcalins ou des méthylates alcalins.
